# EUROPÄISCHE PATENTSCHRIFT

(11) **EP 0 789 684 B1**
(45) Veröffentlichungstag und Bekanntmachung des Hinweises auf die Patenterteilung: **05.04.2000**
(21) Anmeldenummer: 95936538.8
(22) Anmeldetag: 24.10.1995
(51) Int. Cl.: C07C 213/08, C07C 219/06, C07C 219/08, C07C 217/50, C11D 1/835

(54) **VERFAHREN ZUR HERSTELLUNG VON WÄSSRIGEN ESTERQUAT-DISPERSIONEN DURCH QUATERNIERUNG IN GEGENWART VON DISPERGATORGEMISCHEN**
PROCESS FOR THE PRODUCTION OF AQUEOUS ESTERQUAT DISPERSIONS BY QUATERNISATION IN THE PRESENCE OF DISPERSANT MIXTURES
PROCEDE DE PREPARATION DE DISPERSIONS AQUEUSES DE COMPOSES QUATERNAIRES D'ESTER

(30) Priorität: 02.11.1994 DE 4439090
(43) Veröffentlichungstag der Anmeldung: 20.08.1997
(73) Patentinhaber: Cognis Deutschland GmbH, 40589 Düsseldorf (DE)
(72) Erfinder: BIGORRA LLOSAS, Joaquim, E-08203 Sabadell (ES); BRAU BALAQUE, Emili, E-43570 Santa Barbara (ES); PI SUBIRANA, Rafael, E-08400 Granollers (ES)
(86) Internationale Anmeldenummer: EP9504158
(87) Internationale Veröffentlichungsnummer: WO9614290

(56) Entgegenhaltungen:
- EP-A- 0 008 839
- DE-A- 4 243 862
- DE-C- 4 308 794
- US-A- 4 486 195

## Beschreibung

### Gebiet der Erfindung

Die Erfindung betrifft wäßrige, niedrigviskose Esterquat-Dispersionen mit verbessertem Benetzungsvermögen, die man erhält, indem man Fettsäurealkanolaminester in Gegenwart von ausgewählten Dispergatorgemischen quaterniert und anschließend in Wasser einträgt sowie die Verwendung der Dispersionen als oberflächenaktive Mittel.

### Stand der Technik

Quaternierte Fettsäurealkanolaminestersalze, sogenannte "Esterquats", haben in den letzten Jahren als ökotoxikologisch unbedenkliche Rohstoffe für Wäscheweichspüler zunehmend an Bedeutung gewonnen [vgl. O.Ponsati in **C.R. CED-Kongress, Barcelona, 167 (1992)** und R.Puchta in **C.R. CED-Kongress, Sitges, 59 (1993)**].

Zur Herstellung der Esterquats geht man üblicherweise von Fettsäurealkanolaminestern aus, die in Gegenwart von Isopropylalkohol mit geeigneten Alkylierungsmitteln wie beispielsweise Dimethylsulfat quaterniert werden. Dabei resultieren Konzentrate mit etwa 90 Gew.-% Feststoffgehalt im organischen Lösungsmittel, die mit Wasser auf die gewünschte Anwendungskonzentration verdünnt werden können. Die konzentrierten Produkte weisen zwar ausgezeichnete avivierende Eigenschaften auf, sind jedoch vielfach hochviskos.

Aus der **DE-C 4308794** (Henkel/Pulcra) ist ein Verfahren zur Herstellung von Esterquats mit verbesserter Wasserdispergierbarkeit bekannt, bei dem man die Quaternierung in Gegenwart von nichtionischen Tensiden als Dispergatoren bzw. Emulgatoren durchführt. Die resultierenden Produkte sind jedoch fest und müssen als Schuppen in Wasser aufgelöst werden.

Neben der Frage der Anbietungsform weisen Esterquats wie die meisten kationischen Tenside zusätzlich das Problem eines unbefriedigenden Netzvermögens aus. Eine rasche Benetzung der Faser durch das Mittel wäre im Hinblick auf ein hohes Avivagevermögen jedoch besonders wünschenswert. Auch der Trend zu möglichst hochkonzentrierten Produkten läuft dem Bedürfnis nach einem gesteigerten Netzvermögen wegen des erhöhten Kationtensidgehaltes entgegen.

Die Aufgabe der Erfindung hat somit darin bestanden, ein Verfahren zur Herstellung von wäßrigen. niedrigviskosen, radipnetzenden Esterquat-Dispersionen zur Verfügung zu stellen, die frei von den geschilderten Nachteilen sind.

### Beschreibung der Erfindung

Gegenstand der Erfindung ist ein Verfahren zur Herstellung von wäßrigen Esterquat-Dispersionen durch Quaternierung von Fettsäurealkanolaminester in Gegenwart nichtionischer Dispergatoren und Eintragen der Produkte in Wasser, das sich dadurch auszeichnet, daß man als Dispergatoren Gemische einsetzt, enthaltend
(a) Alkoholpolyethylenglycolether der Formel **(I)**,

   R¹O(CH₂CH₂O)ₙH (I)

   in der R¹ für einen linearen oder verzweigten Alkyl- und/oder Alkenylrest mit 6 bis 22 Kohlenstoffatomen und n für Zahlen von 1 bis 10 steht, und
(b) Fettsäureamidoalkylenamine der Formel **(II)**,

   R²CO-NH(Z)NR³R⁴ (II)

   in der R²CO für einen Acylrest mit 12 bis 18 Kohlenstoffatomen und 0 und/oder 1 bis 3 Doppelbindungen und Z für eine lineare oder verzweigte Alkylengruppe mit 2 bis 4 Kohlenstoffatomen und R³ und R⁴ unabhängig voneinander für Wasserstoff oder einen Alkylrest mit 1 bis 4 Kohlenstoffatomen stehen.

Überraschenderweise wurde gefunden, daß die Quaternierung von Fettsäurealkanolaminestern in Gegenwart der genannten Dispergatormischungen nach Eintragen in Wasser zu niedrigviskosen Produkten führt, die ein Netzvermögen gegenüber Baumwolle von unter einer Sekunde aufweisen. Die Erfindung schließt die Erkenntnis ein, daß der nachträgliche Zusatz der genannten Dispergatoren zu nach konventionellen Verfahren hergestellten Esterquats das Benetzungsvermögen der resultierenden Dispersionen nicht oder nur sehr geringfügig verbessert. Ein weiterer Vorteil besteht darin, daß die Dispersionen besonders lagerstabil sind, d.h., auch bei längerer Lagerung homogen bleiben und eine konstante Viskosität aufweisen.

### Esterquats und Fettsäurealkanolaminester

Unter der Bezeichnung Esterquats werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann.

In diesem Zusammenhang sei auf die Internationale Patentanmeldung **WO 91/01295** (Henkel) verwiesen, nach der man Triethanolamin in Gegenwart von unterphosphoriger Säure mit Fettsäu- ren partiell verestert, Luft durchleitet und anschließend mit Dimethylsulfat oder Ethylenoxid quaterniert. Stellvertretend für den umfangreichen Stand der Technik sei an dieser Stelle auf die Druckschriften **US 3915867, US 4370272, EP-A2 0239910, EP-A2 0293955, EP-A2 0295739 und EP-A2 0309052** verwiesen.

Die quaternierten Fettsäuretriethanolaminestersalze folgen der Formel **(IIIa)** 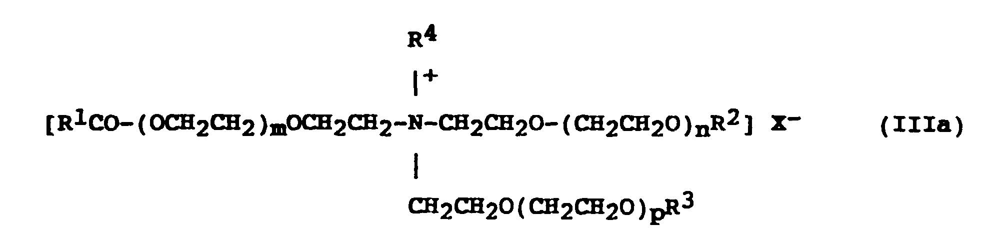 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Im Sinne des erfindungsgemäßen Verfahrens kommen als Ausgangsstoffe für die Alkylierung dementsprechend die nichtquaternierten Fettsäurealkanolaminester der Formel **(III)** in Betracht, 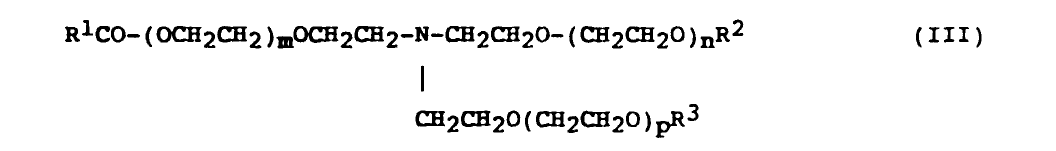 in der R¹CO, R², R³ sowie m, n und p die oben genannten Bedeutungen haben.

Typische Beispiele für Fettsäurealkanolaminester bzw. Esterquats sind Produkte auf Basis von Capronsäure, Caprylsäure, Caprinsäure, Laurinsäure, Myristinsäure, Palmitinsäure, Isostearinsäure, Stearinsäure, ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (Iodzahl 0 bis 40) ab.

Aus anwendungstechnischer Sicht haben sich Fettsäurealkanolaminester der Formel **(III)** bzw. quaternierte Fettsäurealkanolaminestersalze der Formel **(IIIa)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben quaternierten Fettsäuretriethanolaminestersalzen kommen als Esterquats ferner auch quaternierte Estersalze von Fettsäuren mit Diethanolalkylaminen der Formel **(IVa)** in Betracht, 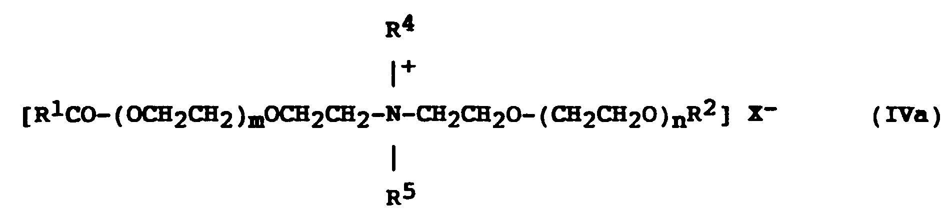 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(Va)** zu nennen, 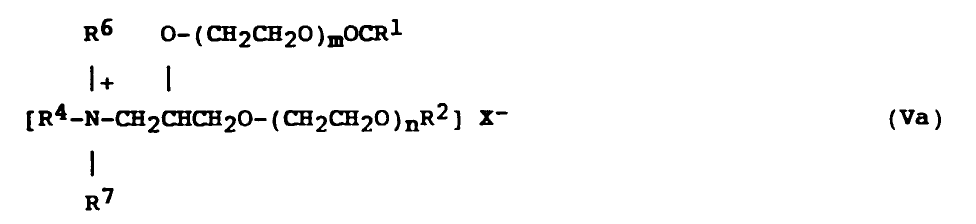 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Den Esterquats der Formel **(IVa)** bzw. **(Va)** liegen wiederum die entsprechenden nichtquaternierten Ester als Ausgangsstoffe zugrunde.

Hinsichtlich der Auswahl der bevorzugten Fettsäuren und des optimalen Veresterungsgrades gelten die für **(III)** bzw. **(IIIa)** genannten Beispiele auch für die Esterquats der Formeln **(IVa)** und **(Va)**.

### Alkoholpolyethylenglycolether

Alkoholpolyethylenglycolether stellen bekannte nichtionische Tenside dar, die durch basenkatalysierte Anlagerung von Ethylenoxid an primäre lineare oder verzweigte Alkohole erhalten werden können.

Im Sinne der Erfindung kommen Ethylenoxid-Addukte an Fettalkohole und vorzugsweise Oxoalkohole in Betracht. Typische Beispiele sind Anlagerungsprodukte von durchschnittlich 1 bis 10, vorzugsweise 3 bis 8 und insbesondere 6 bis 7 Mol Ethylenoxid an Capronalkohol, Caprylalkohol, 2-Ethylhexylalkohol, Caprinalkohol, Laurylalkohol, Isododecylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Die Additionsprodukte können dabei eine konventionelle oder eingeengte Homologenverteilung aufweisen.

Bevorzugt sind Addukte von 6 bis 7 Mol Ethylenoxid an technische Oxoalkohole mit 8 bis 15 Kohlenstoffatomen.

### Fettsäureamidoalkylenamine

Bei den Fettsäureamidoalkylenaminen, die die Dispergatorkomponente (b) ausmachen, handelt es sich um bekannte Kondensationsprodukte von Fettsäuren mit 6 bis 22 und vorzugsweise 12 bis 18 Kohlenstoffatomen mit Diaminen, vorzugsweise Ethylendiamin, Propylendiamin und insbesondere N,N-Dimethylpropylendiamin.

Typische Beispiele sind Amidierungsprodukte von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Ethylendiamin oder Propylendiamin. Besonders bevorzugt ist der Einsatz von Fettsäureamidopropylenaminen und insbesondere Kokos-, Palm- und/oder Talgfettsäureamido-N,N-dimethylpropylenamin.

Üblicherweise können die erfindungsgemäßen Dispergatoren in solchen Mengen eingesetzt werden, daß sich in den resultierenden Quaternierungsprodukten ein Verhältnis von
60 bis 80, vorzugsweise 70 bis 75 Gew.-% Esterquat,
15 bis 25, vorzugsweise 18 bis 22 Gew.-% Komponente (a) und
1 bis 10, vorzugsweise 3 bis 7 Gew.-% Komponente (b)
einstellt, mit der Maßgabe, daß sich die Gewichtsteile zu 100 Gew.-% ergänzen.

Für die Einstellung eines gewünschten Dispergatorgehaltes im Endprodukt ist es erforderlich, die berechneten Mengen der Dispergatorkomponenten (a) und (b) dem Ester vor der Quaternierung zuzusetzen. Die erforderlichen Verhältnisse zu berechnen, bleibt dem Fachmann überlassen, der hierzu nicht erfinderisch tätig werden muß.

### Alkylierung

Die Alkylierung der Fettsäurealkanolaminester kann in an sich bekannter Weise durchgeführt werden. Hierzu wird der Ester vorgelegt und mit dem Alkylierungsmittel - das man üblicherweise in äquimolaren Mengen oder leichtem Unterschuß einsetzt - bei erhöhten Temperaturen gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Betracht. Vorzugsweise betrifft das erfindungsgemäße Verfahren methylquaternierte Esterquats in Form ihrer Chloride oder Methylsulfat-Salze sowie Esterquat-Salze, die mit 1 bis 5 Mol Ethylenoxid quaterniert worden sind.

Die Esterquats werden in Wasser dispergiert, wobei die Dispersionen Feststoffgehalte von 5 bis 35, vorzugsweise 20 bis 30 Gew.-% aufweisen können.

### Gewerbliche Anwendbarkeit

Die nach dem erfindungsgemäßen Verfahren erhältlichen Dispersionen weisen eine niedrige Viskosität auf und sind in rapidnetzend. So weist beispielsweise eine 26 Gew.-%ige Dispersion eines Avivagemittels in Wasser, die 19 Gew.-% Esterquat gemäß Ausführungsbeispiel 1 enthält, eine Netzzeit von weniger als 1 s auf, während ein übliches Handelsprodukt, das bei 26 Gew.-% Feststoff nur 5 Gew.-% des Kationtensids enthält, eine Netzzeit von mehr als 10 s besitzt.

Ein weiterer Gegenstand der Erfindung betrifft daher schließlich die Verwendung der nach dem erfindungsgemäßen Verfahren erhältlichen Dispersionen als oberflächenaktive Mittel. Hierunter sind in erster Linie Avivagemittel, Wäscheweichspülmittel, Wäschevorbehandlungsmittel, Haarshampoos, Haarspülungen, Haarkuren und Haarkonditionierungsmittel zu verstehen.

Die folgenden Beispiele sollen den Gegenstand der Erfindung näher erläutern, ohne ihn darauf einzuschränken.

### Beispiele

### I. Allgemeine Herstellvorschriften

**a) Veresterung.** In einem 1-1-Dreihalskolben mit Rührer, Innenthermometer und Destillationsaufsatz wurden 324 g (1,2 mol) teilgehärtete C_{16/18}-Talg- bzw. Palmfettsäure (Iodzahl = 40), 149 g (1 mol) Triethanolamin und 1,4 g 50 gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Fettsäuretriethanolaminester abgekühlt, der Reaktionsansatz entspannt und unter ständigem Rühren innerhalb von 15 min 1 Liter Luft durchgeleitet.
**b) Quaternierung.** In einem 500-ml-Dreihalskolben mit Rührer, Tropftrichter und Rückflußkühler 75 bis 100 Gewichtsteile des Esters aus (a) zusammen mit 0 bis 20 Gewichtsteilen Isododecanol-6EO (DEHYDOL^{(R)} PID6, Pulcra S.A., Barcelona/ES) und 0 bis 5 Gew.-% C_{16/18}-Talgfettsäureamido-N,N-dimethylpropylenamin vorgelegt und unter Rühren auf 45°C erhitzt. Innerhalb von 2 h wurden 95 Gewichtsteile (bezogen auf den Anteil des Esters) Dimethylsulfat zugetropft. Nach Beendigung der Zugabe wurde die Mischung weitere 2 h bei 60°C gerührt und nichtumgesetztes DMS durch Zusatz von 0,4 g (0,005 mol) Glycin zerstört. Das Esterquat/Dispergator-Gemisch wurde in Wasser dispergiert, wobei eine 26 Gew.-%ige, niedrigviskose Dispersion resultierte.

### II. Anwendungstechnische Beispiele

Das Tauchnetzvermögen verschiedener Esterquat-Typen wurde nach DIN 53 901 bei 20°C in Wasser von 16°d (0,5 g Aktivsubstanz/1) untersucht. Die Bestimmung der Viskosität erfolgte in einem Brookfield-RVT-Viskosimeter bei 23°C, Spindel 2 und 20 Upm. Die Dispergierbarkeit wurde subjektiv bestimmt. Die Skala reichte von (--) = "nicht dispergierbar" bis (+++) = "sehr leicht dispergierbar". Die Ergebnisse sind in Tabelle 1 zusammengefaßt.

Die Beispiele und Vergleichsbeispiele zeigen, daß die erfindungsgemäße Aufgabe, rapidnetzende, niedrigviskose Esterquats zur Verfügung zu stellen, ausschließlich durch Einsatz der erfindungsgemäßen Dispergatormischung in der Quaternierung erreicht wird. Ein nachträglicher Zusatz der Komponenten (a) und (b) gemäß Vergleichsbeispiel V5 führt zu signifikant schlechteren Ergebnissen.

**Tabelle 1**

| Netzvermögen und Viskosität | | | | | | | |
|---|---|---|---|---|---|---|---|
| **Bsp.** | **FSB** | **C[E] GT** | **c[N] GT** | **c[A] GT** | **Netz. s** | **Viskos. MPas*s** | **Dis.** |
| 1 | Palm | 75 | 20 | 5 | < 1 | 850 | +++ |
| 2 | Talg | 75 | 20 | 5 | < 1 | 900 | +++ |
| V1* | Palm | 100 | 0 | 0 | 10 | 3900 | -- |
| V2* | Talg | 100 | 0 | 0 | 5 | 4000 | - |
| V3 | Talg | 80 | 20 | 0 | 3 | 3900 | - |
| V4* | Talg | 95 | 0 | 5 | 10 | 1200 | + |
| V5 | Talg | 75 | 20 | 5 | 9 | 3500 | + |
| Legende: FSB = Fettsäurebasis c[E] = Anteil Ester in Gewichtsteilen c[N] = Anteil Niotensid c[A] = Anteil Amidoamin * = Versuche wurden in Isopropylalkohol als Lösungsmittel durchgeführt Netz. = Netzzeit Visk. = Viskosität Dis. = Dispergierbarkeit | | | | | | | |

## Patentansprüche

1. Verfahren zur Herstellung von wäßrigen Esterquat-Dispersionen durch Quaternierung von Fettsäurealkanolaminester in Gegenwart nichtionischer Dispergatoren und Eintragen der Produkte in Wasser, **dadurch gekennzeichnet**, daß man als Dispergatoren Gemische einsetzt, enthaltend
(a) Alkoholpolyethylenglycolether der Formel **(I)**,
R¹O(CH₂CH₂O)ₙH (I)
in der R¹ für einen linearen oder verzweigten Alkyl- und/oder Alkenylrest mit 6 bis 22 Kohlenstoffatomen und n für Zahlen von 1 bis 10 steht, und
(b) Fettsäureamidoalkylenamine der Formel **(II)**,
R²CO-NH(Z)NR³R⁴ (II)
in der R²CO für einen Acylrest mit 12 bis 18 Kohlenstoffatomen und 0 und/oder 1 bis 3 Doppelbindungen und Z für eine lineare oder verzweigte Alkylengruppe mit 2 bis 4 Kohlenstoffatomen und R³ und R⁴ unabhängig voneinander für Wasserstoff oder Alkylreste mit 1 bis 4 Kohlenstoffatomen stehen.

2. Verfahren nach Anspruch 1, **dadurch gekennzeichnet**, daß man Fettsäurealkanolaminester der Formel **(III)** einsetzt, 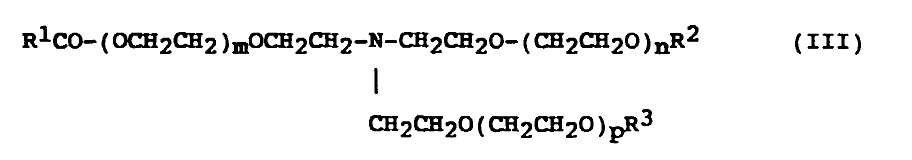 in der R¹CO für einen Acylrest mit 6 bis 22 Kohlenstoffatomen, R² und R³ unabhängig voneinander für Wasserstoff oder R¹CO, m, n und p in Summe für 0 oder Zahlen von 1 bis 12 steht.

3. Verfahren nach den Ansprüchen 1 und 2, **dadurch gekennzeichnet**, daß man als Dispergatorkomponente (a) Anlagerungsprodukte von durchschnittlich 3 bis 8 Mol Ethylenoxid an Oxoalkohole mit 8 bis 15 Kohlenstoffatomen einsetzt.

4. Verfahren nach den Ansprüchen 1 bis 3, **dadurch gekennzeichnet**, daß man als Dispergatorkomponente (b) Fettsäureamido-N,N-dimethylpropylenamine einsetzt.

5. Verfahren nach den Ansprüchen 1 bis 4, **dadurch gekennzeichnet**, daß man die Dispergatormischung in solchen Mengen einsetzt, daß sich in den resultierenden Quaternierungsprodukten ein Verhältnis von
60 bis 80 Gew.-% Esterquat,
15 bis 25 Gew.-% Komponente (a) und
1 bis 10 Gew.-% Komponente (b)
einstellt, mit der Maßgabe, daß sich die Gewichtsteile zu 100 Gew.-% ergänzen.

6. Verfahren nach den Ansprüchen 1 bis 5, **dadurch gekennzeichnet**, daß man Alkylierungsmittel ausgewählt aus der Gruppe, die von Alkylhalogeniden, Dialkylsulfaten und Ethylenoxid gebildet wird, einsetzt.

7. Verwendung der wäßrigen Dispersionen nach den Ansprüchen 1 bis 6 als oberflächenaktive Mittel.

## Claims

1. A process for the production of aqueous esterquat dispersions by quaternization of fatty acid alkanolamine esters in the presence of nonionic dispersants and introduction of the products into water, characterized in that the dispersants used are mixtures containing
(a) alcohol polyethylene glycol ethers corresponding to formula (I):
R¹O(CH₂CH₂O)ₙH (I)
in which R¹ is a linear or branched alkyl and/or alkenyl group containing 6 to 22 carbon atoms and n is a number of 1 to 10, and
(b) fatty acid amidoalkylene amines corresponding to formula (II):
R²CO-NH(Z)NR³R⁴ (II)
in which R²CO is an acyl group containing 12 to 18 carbon atoms and 0 and/or 1 to 3 double bonds and Z is a linear or branched alkylene group containing 2 to 4 carbon atoms and R³ and R⁴ independently of one another represent hydrogen or alkyl groups containing 1 to 4 carbon atoms.

2. A process as claimed in claim 1, characterized in that fatty acid alkanolamine esters corresponding to formula (III): 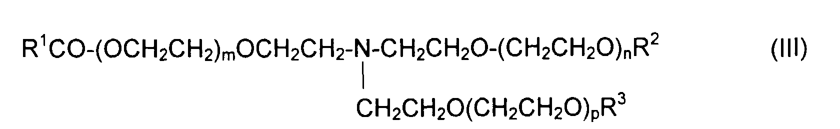 in which R¹CO is an acyl group containing 6 to 22 carbon atoms, R² and R³ independently of one another represent hydrogen or R¹CO and m, n and p together stand for 0 or numbers of 1 to 12,
are used.

3. A process as claimed in claims 1 and 2, characterized in that products of the addition of on average 3 to 8 moles of ethylene oxide onto oxoalcohols containing 8 to 15 carbon atoms are used as dispersant component (a).

4. A process as claimed in claims 1 to 3, characterized in that fatty acid amido-N,N-dimethyl propylene amines are used as dispersant component (b).

5. A process as claimed in claims 1 to 4, characterized in that the dispersant mixture is used in such quantities that a ratio of
60 to 80% by weight of esterquat,
15 to 25% by weight of component (a) and
1 to 10% by weight of component (b)
- with the proviso that the parts by weight add up to 100% by weight -
is established in the resulting quaternization products.

6. A process as claimed in claims 1 to 5, characterized in that alkylating agents selected from the group consisting of alkyl halides, dialkyl sulfates and ethylene oxide are used.

7. The use of the aqueous dispersions according to claims 1 to 6 as surface-active compositions.

## Revendications

1. Procédé de fabrication de dispersions aqueuses d'ester-quats par quaternisation d'esters d'alcanolamines d'acides gras en présence de dispersants non ioniques et par l'introduction des produits dans l'eau,
caractérisé en ce qu'
on utilise comme dispersants des mélanges contenant
(a) des polyéthylène glycoléthers d'alcools de formule (I) :
R¹O(CH₂CH₂O)ₙH (I)
dans laquelle R¹ représente un radical alkyle et/ou alcényle linéaire ou ramifié ayant de 6 à 22 atomes de carbone et n représente des nombres de 1 à 10 et
(b) des amidoalkylènamines d'acides gras de formule (II),
R²CO-NH(Z)NR³R⁴ (II)
dans laquelle R²CO représente un radical acyle ayant de 12 à 18 atomes de carbone et de 0 et/ou 1 à 3 doubles liaisons, Z représente un groupe alkylène linéaire ou ramifié ayant 2 ou 4 atomes de carbone, et R³ et R⁴ représentent indépendamment l'un de l'autre un hydrogène ou un radical alkyle ayant de 1 à 4 atomes de carbone.

2. Procédé selon la revendication 1,
caractérisé en ce qu'
on utilise des alcanolaminesters d'acides gras de formule (III) : 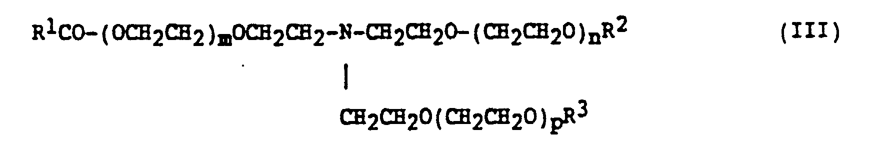 dans laquelle R¹CO représente un radical acyle ayant 6 à 22 atomes de carbone, R² et R³ représentent indépendamment l'un de l'autre un hydrogène ou R¹CO, m, n et p valent 0 ou des nombres de 1 à 12.

3. Procédé selon les revendications 1 et 2,
caractérisé en ce qu'
on utilise comme composants dispersants (a) des produits de fixation d'en moyenne 3 à 8 moles d'oxyde d'éthylène sur des oxoalcools ayant 8 à 15 atomes de carbone.

4. Procédé selon les revendications 1 à 3,
caractérisé en ce qu'
on utilise comme composant dispersant (b) des amido-N,N-diméthylpropylènamines d'acides gras.

5. Procédé selon les revendications 1 à 4,
caractérisé en ce qu'
on utilise le mélange de dispersant en des quantités telles que l'on établit dans les produits de quaternisation résultants un rapport de
60 à 80 % d'esterquats,
15 à 25 % en poids de composant (a) et
1 à 10 % en poids de composant (b),
sous réserve que les parties en poids se complètent à 100 % en poids.

6. Procédé selon les revendications 1 à 5,
caractérisé en ce qu'
on utilise des agents alkylants choisis dans le groupe formé par les halogénures d'alkyle, les sulfates de dialkyle et l'oxyde d'éthylène.

7. Utilisation des dispersions aqueuses selon les revendications 1 à 6 comme agents tensioactifs.
